# EUROPEAN PATENT SPECIFICATION

(11) **EP 2 912 633 B1**
(45) Date of publication and mention of the grant of the patent: **28.03.2018**
(21) Application number: 13779847.6
(22) Date of filing: 22.10.2013
(51) Int. Cl.: G06T 7/30

(54) **SIMULATION OF OBJECTS IN AN ATLAS AND REGISTRATION OF PATIENT DATA CONTAINING A SPECIFIC STRUCTURE TO ATLAS DATA**
SIMULATION VON OBJEKTEN IN EINEM ATLAS UND REGISTRIERUNG VON PATIENTENDATEN MIT SPEZIFISCHER STRUKTUR IN ATLASDATEN
SIMULATION D'OBJETS DANS UN ATLAS ET ENREGISTREMENT DE DONNÉES DE PATIENT CONTENANT UNE STRUCTURE SPÉCIFIQUE SUR DES DONNÉES D'ATLAS

(30) Priority: 26.10.2012 WO PCT/EP2012/071239; 26.10.2012 WO PCT/EP2012/071241; 30.09.2013 WO PCT/EP2013/070331
(43) Date of publication of application: 02.09.2015
(73) Proprietor: Brainlab AG, 81829 München (DE)
(72) Inventor: BLUMHOFER, Andreas, 85579 Neubiberg (DE); VÁRKUTI, Bálint, 81541 Munich (DE); FROMMERT, Mona, 81541 Munich (DE)
(74) Representative: Schwabe - Sandmair - Marx
(86) International application number: PCT/EP2013/072009
(87) International publication number: WO 2014/064066

(56) References cited:
- ZACHARAKI E I ET AL: "ORBIT: A Multiresolution Framework for Deformable Registration of Brain Tumor Images", IEEE TRANSACTIONS ON MEDICAL IMAGING, IEEE SERVICE CENTER, PISCATAWAY, NJ, US, vol. 27, no. 8, 1 August 2008 (2008-08-01) , pages 1003-1017, XP011224706, ISSN: 0278-0062, DOI: 10.1109/TMI.2008.916954
- ZACHARAKI E I ET AL: "Non-diffeomorphic registration of brain tumor images by simulating tissue loss and tumor growth", NEUROIMAGE, ACADEMIC PRESS, ORLANDO, FL, US, vol. 46, no. 3, 1 July 2009 (2009-07-01), pages 762-774, XP027453032, ISSN: 1053-8119, DOI: 10.1016/J.NEUROIMAGE.2009.01.051 [retrieved on 2009-02-05]
- BACHCUADRA M ET AL: "Atlas-Based Segmentation of Pathological MR Brain Images Using a Model of Lesion Growth", IEEE TRANSACTIONS ON MEDICAL IMAGING, IEEE SERVICE CENTER, PISCATAWAY, NJ, US, vol. 23, no. 10, 1 October 2004 (2004-10-01), pages 1301-1314, XP011119648, ISSN: 0278-0062, DOI: 10.1109/TMI.2004.834618
- PRASTAWA M ET AL: "Simulation of brain tumors in MR images for evaluation of segmentation efficacy", MEDICAL IMAGE ANALYSIS, OXFORD UNIVERSITY PRESS, OXOFRD, GB, vol. 13, no. 2, 1 April 2009 (2009-04-01), pages 297-311, XP026028252, ISSN: 1361-8415, DOI: 10.1016/J.MEDIA.2008.11.002 [retrieved on 2008-12-03]

## Description

### FIELD OF THE INVENTION

The present invention is directed to the simulation of objects, such as pathological objects, in an atlas, such as a tissue-class atlas, using meta-information and the registration of patient data containing a specific structure, such as for example a pathological structure, e.g. a tumor, to atlas data which does not contain such a specific structure or pathological structure.

### BACKGROUND OF THE INVENTION

Elastic registration of patient images containing pathologies, such as tumors, to an atlas is a challenge, since the pathology is not contained in the atlas. The mass effect of a tumor is usually too big for standard algorithms to yield satisfactory results. The simulation of pathological objects and in particular the simulation of tumors in brain atlases has been addressed by several specific studies in order to improve the registration of pathological patient images to the atlas (see for example IEEE Trans Biomed Eng. 2008 March; 55(3): 1233-1236, and Proc. SPIE 6914, Medical Imaging 2008: Image Processing, 69140K and references therein). The simulation of tumors is usually done by placing a seed into the tumor center and by simulating the tumor mass effect using a biomechanical model of soft tissue deformation or a simple radial growth model.

### PRIOR ART

US 2013/0063434 A1 discloses a method and system of compensation for intra-operative organ shift of a living subject including the steps of: generating a first geometric surface of the organ of the living subject from intra-operatively acquired images of the organ of the living subject, constructing an atlas of organ deformations of the living subject from preoperatively acquired organ images, generating a second geometric surface of the organ from the atlas of organ deformations, aligning the second geometric surface of the organ to the first geometric surface of the organ of the living subject to determine at least one difference between a point of the first geometric surface and a corresponding point of the second geometric surface of the organ of the living subject, which is related to organ shift, and compensating for the intra-operative organ shift.

WO 2008/041125 A2 discloses systems for and methods of registering a deformable image with a reference image subject to a spatially variant flexibility model or a non-Gaussian smoothing model, allowing for the consideration of differences between the ways in which structures of interest may move within a patient.

CN 102262699 A discloses a soft tissue deformation simulation method based on coupling of mesh-free Galerkin and mass spring.

ZACHARAKI E. I. ET AL: "ORBIT: A Multiresolution Framework for Deformable Registration of Brain Tumor Images", IEEE TRANSACTIONS ON MEDICAL IMAGING, IEEE SERVICE CENTER, PISCATAWAY, NJ, US, vol. 27, no. 8, 1 August 2008, pages 1003-1017, XP011224706 discloses a deformable registration method for registering a normal brain atlas with images of brain tumor patients. The registration is facilitated by first simulating the tumor mass effect in the normal atlas in order to create an atlas image that is as similar as possible to the patient's image. An optimization framework is used to optimize the location of tumor seed as well as other parameters of the tumor growth model, based on the pattern of deformation around the tumor region. In particular, the optimization is implemented in a multiresolution and hierarchical scheme, and it is accelerated by using a principal component analysis (PCA)-based model of tumor growth and mass effect, trained on a computationally more expensive biomechanical model. Validation on simulated and real images shows that the proposed registration framework, referred to as ORBIT (optimization of tumor parameters and registration of brain images with tumors), outperforms other available registration methods particularly for the regions close to the tumor, and it has the potential to assist in constructing statistical atlases from tumor-diseased brain images.

ZACHARAKI E. I. ET AL: "Non-diffeomporphic registration of brain tumor images by simulating tissue loss and tumor growth", NEUROIMAGE, ACADEMIC PRESS, ORLANDO, FL, US, vol. 46, no. 3, 1 July 2009, pages 762-774, XP 027453032 discloses a spatial normalization of MR images from patients with brain tumors in a common stereotaxic space, aiming to pool data from different patients into a common space in order to perform group analyses.

BACHCUADRA M. ET AL: "Atlas-Based Segmentation of Pathological MR Brain Images Using a Model of Lesion Growth", IEEE TRANSACTIONS ON MEDICAL IMAGING, IEEE SERVICE CENTER, PISCATAWAY, NJ, US, vol. 23, no. 10, 1 October 2004, pages 1301-1314, XPO11119648 discloses a method for brain atlas deformation in the presence of large space-occupying tumors, based on an a priori model of lesion growth that assumes radial expansion of the lesion from its starting point.

PRASTAWA M. ET AL: "Simulation of brain tumors in MR images for evaluation of segmentation efficacy", MEDICAL IMAGE ANALYSIS, OXFORD UNIVERSITY PRESS, OXOFRD, GB, vol. 13, no. 2, 1 April 2009, pages 297-311, XP026028252 presents a system that combines physical and statistical modelling to generate synthetic multi-modal 3D brain MRI with tumor and edema. Pathology is synthesized in multi-modal MRI and diffusion tensor imaging by simulating mass effect, warping and destruction of white matter fibers, and infiltration of brain tissues by tumor cells.

### OBJECTS OF THE INVENTION

It is an object of the present invention to simulate the change or growth of an object, for example of a tumor, within an entity, such as a body or a part of a body, e.g. a brain, in a representation or atlas representation of the entity. It is another object to simulate or perform the registration of patient data containing a specific object, for example a specific structure or pathological structure, to atlas data.

These objects are achieved by the subject-matter of the independent claims. The dependent claims are directed to advantageous embodiments. Different advantageous features can be combined in accordance with the invention wherever technically sensible and feasible. A feature of one embodiment which is functionally identical or similar to a feature of another embodiment can in particular replace the latter feature. A feature of an embodiment which supplements a function of another embodiment can in particular be added to the other embodiment.

### DEFINITIONS

Hereinafter an explanation and a definition of terms used to describe the invention is given.

### Atlas (data)

An atlas or atlas data typically consists of a plurality of generic models of objects, wherein the generic models of the objects together form a complex structure. The atlas of a brain, for example, can comprise the telencephalon, the cerebellum, the diencephalon, the pons, the mesencephalon and the medulla as the objects which together make up the complex structure. The atlas of a femur, for example, can comprise the head, the neck, the body, the greater trochanter, the lesser trochanter and the lower extremity as objects which together make up the complete structure.

The atlas or anatomical atlas can describe the general anatomical structure of either the complete body of a patient model or an object in the patient model or parts of or a plurality of objects in the patient model, which in particular have a defined positional relationship with respect to each other. An object within the atlas can comprise one or more anatomical elements. The atlas can be a two-dimensional or a three-dimensional (static) atlas or a time-dependent two-dimensional or three-dimensional atlas (referred to as 4D atlas).

An application of such an atlas can be the segmentation of medical images, in which the atlas is matched to medical image data, and the image data is compared with the matched atlas in order to assign a point (a pixel or voxel) of the image data to an object of the matched atlas, thereby segmenting the image data into objects.

### Imaging methods

In the field of medicine, imaging methods (also called imaging modalities and/or medical imaging modalities) are used to generate image data (for example, two-dimensional or three-dimensional image data) of anatomical structures (such as the brain, soft tissues, bones, organs, etc.) of the human body. The term "medical imaging methods" is understood to mean (advantageously apparatus-based) imaging methods (so-called medical imaging modalities and/or radiological imaging methods) such as for instance computed tomography (CT) and cone beam computed tomography (CBCT, in particular volumetric CBCT), x-ray tomography, magnetic resonance tomography (MRT or MRI), conventional x-ray, sonography and/or ultrasound examinations, and positron emission tomography. Analytical devices in particular are used to generate the image data in apparatus-based imaging methods. The imaging methods are in particular used for medical diagnostics, to analyse the anatomical body in order to generate images which are described by the image data. The imaging methods are also in particular used to detect pathological changes in the human body, such as e.g. tumors. However, some of the changes in the anatomical structure, in particular the pathological changes in the structures (tissue), may not be detectable and in particular may not be visible in the images generated by the imaging methods. A tumor represents an example of a change in an anatomical structure. If the tumor grows, it may then be said to represent an expanded or specific anatomical structure. This expanded or specific anatomical structure may not be detectable; in particular, only a part of the expanded or specific anatomical structure may be detectable. Primary/high-grade brain tumors are for example usually visible on MRI scans when contrast agents are used to infiltrate the tumor. MRI scans represent an example of an imaging method. In the case of MRI scans of such brain tumors, the signal enhancement in the MRI images (due to the contrast agents infiltrating the tumor) is considered to represent the solid tumor mass. Thus, the tumor is detectable and in particular discernible in the image generated by the imaging method. In addition to these tumors, referred to as "enhancing" tumors, it is thought that approximately 10% of brain tumors are not discernible on a scan and are in particular not visible to a user looking at the images generated by the imaging method.

### Data processing method

The methods in accordance with the invention are in particular data processing methods. A data processing method is preferably performed using technical means, in particular a computer. The data processing method is preferably constituted to be executed by or on a computer, in particular it is executed by or on the computer. In particular, all the steps or merely some of the steps (i.e. less than the total number of steps) of the method in accordance with the invention can be executed by a computer. The computer in particular comprises a processor and a memory in order to process the data, in particular electronically and/or optically. The calculating and simulating steps described are in particular performed by a computer. Determining steps or calculating step, such as segmenting or performing a fusion, are in particular steps of determining data within the framework of the technical data processing method, in particular within the framework of a program.

A computer is in particular any kind of data processing device, in particular electronic data processing device. A computer can be a device which is generally thought of as such, for example desktop PCs, notebooks, netbooks, etc., but can also be any programmable apparatus, such as for example a mobile phone or an embedded processor. A computer can in particular comprise a system (network) of "sub-computers", wherein each sub-computer represents a computer in its own right. The term "computer" includes a cloud computer, in particular a cloud server. The term "cloud computer" includes a cloud computer system which in particular comprises a system of at least one cloud computer and in particular a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. In particular, the term "cloud" is used in this respect as a metaphor for the Internet (world wide web). In particular, the cloud provides computing infrastructure as a service (IaaS). The cloud computer can function as a virtual host for an operating system and/or data processing application which is used to execute the method of the invention. The cloud computer is for example an elastic compute cloud (EC2) as provided by Amazon Web Services™. A computer in particular comprises interfaces in order to receive or output data and/or perform an analogue-to-digital conversion.

The data are in particular data which represent physical properties and/or are generated from technical signals. The technical signals are in particular generated by means of (technical) detection devices (such as for example devices for detecting marker devices) and/or (technical) analytical devices (such as for example devices for performing imaging methods), wherein the technical signals are in particular electrical or optical signals. The technical signals in particular represent the data received or outputted by the computer.

The computer is preferably operatively coupled to a display device which allows to display information outputted by the computer e.g. to a user. An example of a display device is an augmented reality device (also called augmented reality glasses) which may be used as goggles for navigating. A specific example of such augmented reality glasses is Google Glass (trademark of Google Inc.). An augmented reality device may be used to both input information into the computer by user interaction and to display information outputted by that computer.

### Acquiring Data

The expression "acquiring data" in particular encompasses (within the framework of a data processing method) the scenario in which the data are determined by the data processing method or program. Determining data in particular encompasses measuring physical quantities and transforming the measured values into data, in particular digital data, and/or computing the data by means of a computer and in particular within the framework of the method in accordance with the invention. The meaning of "acquiring data" also in particular encompasses the scenario in which the data are received or retrieved by the data processing method or program, for example from another program, a previous method step or a data storage medium, in particular for further processing by the data processing method or program. The expression "acquiring data" can therefore also for example mean waiting to receive data and/or receiving the data. The received data can for example be inputted via an interface. The expression "acquiring data" can also mean that the data processing method or program performs steps in order to (actively) receive or retrieve the data from a data source, for instance a data storage medium (such as for example a ROM, RAM, database, hard drive, etc.), or via the interface (for instance, from another computer or a network). The data can be made "ready for use" by performing an additional step before the acquiring step. In accordance with this additional step, the data are generated in order to be acquired. The data are in particular detected or captured (for example by an analytical device). Alternatively or additionally, the data are inputted in accordance with the additional step, for instance via interfaces. The data generated can in particular be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, in particular determining data, does not involve a surgical step and in particular does not involve a step of treating a human or animal body using surgery or therapy. In order to distinguish the different data used by the present method, the data are denoted (i.e. referred to) as "patient data", "atlas data", "registered atlas data" and the like and are defined in terms of the information which they describe.

### (Image) fusion

(Image) fusion can be elastic (image) fusion and/or rigid (image) fusion. In case of rigid (image) fusion the relative position between the pixels or voxels of data or an image (2D or 3D) is fixed while in case of elastic image fusion, the relative positions are allowed to change.

Elastic fusion transformations (for example, elastic image fusion transformations) are in particular designed to enable a seamless transition from one data set (for example a first data set such as for example a first image) to another data set (for example a second data set such as for example a second image). The transformation is in particular designed such that one of the first and second data sets (images) is deformed, in particular in such a way that corresponding structures (in particular, corresponding image elements) are arranged at the same position as in the other of the first and second images. The deformed (transformed) image which is transformed from one of the first and second images is in particular as similar as possible to the other of the first and second images. Preferably, (numerical) optimisation algorithms are applied in order to find the transformation which results in an optimum degree of similarity. The degree of similarity is preferably measured by way of a measure of similarity (also referred to in the following as a "similarity measure"). The parameters of the optimisation algorithm are in particular vectors of a deformation field. These vectors are determined by the optimisation algorithm which results in an optimum degree of similarity. Thus, the optimum degree of similarity represents a condition, in particular a constraint, for the optimisation algorithm. The bases of the vectors lie in particular at voxel positions of one of the first and second images which is to be transformed, and the tips of the vectors lie at the corresponding voxel positions in the transformed image. A plurality of these vectors are preferably provided, for instance more than twenty or a hundred or a thousand or ten thousand, etc. Preferably, there are (other) constraints on the transformation (deformation), in particular in order to avoid pathological deformations (for instance, all the voxels being shifted to the same position by the transformation). However, a parallel shift of an object may be allowed. These constraints include in particular the constraint that the transformation is regular, which in particular means that a Jacobian determinant calculated from a matrix of the deformation field (in particular, the vector field) is larger than zero, and the constraint that the transformed (deformed) image is not self-intersecting and in particular that the transformed (deformed) image does not comprise faults and/or ruptures. The constraints include in particular the constraint that if a regular grid is transformed simultaneously with the image and in a corresponding manner, the grid is not allowed to interfold at any of its locations. The optimising problem is in particular solved iteratively, in particular by means of an optimisation algorithm which is in particular a first-order optimisation algorithm, in particular a gradient descent algorithm. Other examples of optimisation algorithms include optimisation algorithms which do not use derivations such as the downhill simplex algorithm or algorithms which use higher-order derivatives such as Newton-like algorithms. The optimisation algorithm preferably performs a local optimisation. If there are a plurality of local optima, global algorithms such as simulated annealing or generic algorithms can be used. In the case of linear optimisation problems, the simplex method can for instance be used.

In the steps of the optimisation algorithms, the voxels are in particular shifted by a magnitude in a direction such that the degree of similarity is increased. This magnitude is preferably less than a predefined limit, for instance less than 1/10 or 1/100 or 1/1000 of the diameter of the image, and in particular about equal to or less than the distance between neighbouring voxels. Large deformations can be implemented, in particular due to a high number of (iteration) steps.

The determined elastic fusion transformation can in particular be used to determine a degree of similarity (or similarity measure, see above) between the first and second data sets (first and second images). To this end, the deviation between the elastic fusion transformation and an identity transformation is determined. The degree of deviation can for instance be calculated by determining the difference between the determinant of the elastic fusion transformation and the identity transformation. The higher the deviation, the lower the similarity, hence the degree of deviation can be used to determine a measure of similarity.

A measure of similarity can in particular be determined on the basis of a determined correlation between the first and second data sets. The similarity measure (mostly the cross-correlation) can be used in order to determine the correct elastic fusion transformation iteratively. In order to reach this goal, the cross-correlation between the two images after the current elastic fusion transformation has been applied to one of them is computed, and the elastic fusion transformation is adapted to optimize the value of the cross-correlation.

### Computer

The method in accordance with the invention is preferably at least partly executed by a computer, i.e. all the steps or merely some of the steps (i.e. less than the total number of steps) of the method in accordance with the invention can be executed by a computer.

### Metadata / meta-information

The term metadata or meta-information (preferably used as an equivalent expression for metadata) refers in general to "data about data". In a more specific aspect, the term refers to descriptive metadata specifying a feature, such as a behavior or property, of a structure, tissue or body part. Metadata or meta-information can be data or information stored in an atlas as a set of rules for a given object or tissue type or for a given absolute or relative position with respect to other anatomical structures. Metadata can optionally include the probability for one or more given rules to occur, such as the likelihood for the rule (a tumor grows radially) to occur, depending on the properties of the tissue or structures the tumor is in or surrounded by.

Meta-information can be used for simulating, estimating or predicting the future change or growth of a structure or tissue, such as a tumor, within other tissue.

The meta-information or metadata can be obtained by performing a statistical analysis over many different objects or patients, and by optionally converting the insights gained from this analysis into rules being preferably simplified rules specifying for example a predicted change (e.g. tumor growth) and optionally the probability of this rule to occur. The meta-information can be added to an atlas to enable a simulation or prediction of future changes to occur, such as the simulation of the change or growth of a pathological structure contained in patient images matched to a (tissue-class) atlas. The meta-information may contain in particular information about the interaction between the pathological object and the tissue-classes of the tissue-class atlas, so the invention is mostly useful for a tissue-class atlas. Such a modified (universal) atlas provides the capability to compute modified atlases of an arbitrary sequence or to co-register different sequences of a patient onto a single universal atlas containing the pathology.

### SUMMARY OF THE INVENTION

The present invention is directed to a data processing method for registering patient data containing a specific or pathological object or pathological structure, such as e.g. a tumor or an edema, to atlas data containing no such specific or pathological structure or object. The method is performed by a computer and comprises the following steps: patient data is acquired, preferably by an imaging method as set forth above, which comprises an information or anatomical information of the patient including the normal or non-special (healthy) as well as the special or pathological structure of the patient. Patient data can be data covering only a specific region of the patient, such as for example the head or the brain only, or can encompass larger structures or even the whole patient body.

The patient data is at least partially segmented to define for example an estimate of or the exact boundaries of the special or pathological structure within the normal or healthy structure, such as the extension or dimensions of a tumor. This provides the possibility to remove, for a later described fusion process, the pathological structure from the patient data. It also provides the tumor position in the atlas by applying the fusion mapping (which matches the patient onto the atlas) to the tumor object. In addition, further segmentation steps can be performed on the patient data to obtain sub-structures or tissue-structures within the patient data used for the below described fusion.

Atlas data related or corresponding to the acquired patient data or at least a part of the acquired patient data is acquired, which atlas data is or can be provided e.g. from a data base.

A fusion or image fusion of the acquired patient data excluding the pathological structure, that is the patient data minus the segmented pathological structure, or simply the patient data with masked out pathological structure, and the atlas data is performed to obtain registered atlas data. The pathological structure can thus be masked out in order not to contribute to a similarity measure for the fusion or mapping.

The change or growth of a simulated specific or pathological structure is simulated within the registered atlas data (up to now not having data related to or describing the specific or pathological structure) using the registered atlas data and meta-information about objects of the atlas data being adjacent to or surrounding the area of the simulated pathological structure to obtain registered atlas data containing a simulated grown pathological structure.

If for example the tumor is at or touches the Dura Mater, a seed for the first simulation step of the tumor growth can e.g. be set in the middle of the boundary surface between tumor and Dura Mater (in this case the tumor is transferred first with a registration step to the atlas to be an "atlas registered" tumor). Otherwise, the seed is placed in the middle of the tumor.

The simulation step can be performed for all kinds of pathological or foreign objects, such as tumors, edema and struma. In addition, the above mentioned method can be performed to analyze or simulate the interaction of structures or specific objects, such as implants, insulin pumps, catheters, etc. with tissue. In this case, the specific or foreign or pathological structure can be an external (non-tissue) object which will or may be incorporated into surrounding tissue by said tissue growing around or into this object.

The above mentioned fusion step can be a rigid fusion, an elastic fusion or a per se known combination of a rigid fusion (preferably performed first) and an elastic fusion (preferably performed after performing the rigid fusion).

Depending on pre-defined meta-information of tissue being adjacent to or surrounding the specific object, a change and/or shift of such tissue can be simulated. Change can e.g. be from healthy tissue data to pathological tissue data, i.e. a transformation of the tissue class. Shift can e.g. be in one or more predetermined direction(s) specified by the meta-information or e.g. by a preferably simple or simplified rule, which rule can be associated as meta-information to specific tissue or tissue-classes or specific relative locations (e.g. tissue of the same tissue-class is distinguished by its positions, such as touching Dura Mater or not) and/or to the type of the specific object (e.g. is a tumor infiltrative or not; is the tumor larger or smaller than a predefined value, e.g. 30cm³).

The above method can be performed iteratively. After having performed a simulation of the change of tissue and/or the pathological structure, such as a tumor mass-effect, the mapping corresponding to the mass-effect can be concatenated with the registration mapping. Subsequently, a new registration can be performed, in which the tumor (again) is masked out such that it does not contribute to the similarity measure used to determine the registration mapping. The fusion and simulation steps can then be repeated until a desired accuracy is reached.

In most cases the object (tumor) in the atlas is simply obtained by transforming the object segmented in the patient with the registration. In particular cases of infiltrative tumors, a segmentation of the simulated object in the atlas can or probably need to be performed, but this will be an exception and is not mandatory.

The above method steps can also be used to simulate (in the sense of predict) a (future) growth or change of a specific object, such as a tumor.

Thus, according to the present invention, the simulation of the change or past and/or future growth of a specific structure, such as the simulation of tumors, can exploit all possible information about the object that can be used to obtain optimal simulation results. This (meta-) information can include information about where tumors typically start growing, in which direction they usually grow, how fast they grow, etc. This information can for example be added as meta-information to an atlas or atlas data and can be refined to be added to specific tissue classes represented by the atlas.

Thus, for example in the Cerebrum, meta-information can be stored to simulate the growth of the tumor by placing the tumor seed in the center of the tumor. For tumors (in particular meningiomas) in the sub-tentorial region, a more sophisticated model for placing the tumor seed can be generated or stored as meta-data. The Dura Mater represents a hard limit for tumor growth and in the case of meningiomas, the tumor even originates in the Dura Mater, and then usually grows away from the Dura Mater into a specific region. Placing the tumor seed into the center of the tumor would result in a wrong shift of the surrounding tissue. According to the present invention, meta-information can be provided to specify within a atlas data or registered atlas data a more accurate or better location of the tumor seed being the starting point of simulating the growth of the tumor.

In addition, the present invention can also be used to simulate the relaxation of surrounding tissue, in particular the brainstem, after tumor resection. A prediction of this effect is crucial for post-operative treatment planning, and can also be used to plan an optimal operation strategy. In the case of simulating the relaxation of surrounding tissue after tumor resection, the specific object data segmented in the patient data is the pathological structure to be removed, e.g. the tumor. The "growth" of the simulated specific object can then be a negative "growth", i.e. the simulation of a shrinking process of the area of the removed structure and/or the simulation of a relaxation process of surrounding tissue. In this case, meta-information can be associated to tissue specifying such relaxation (or expanding) properties of respective different tissue classes.

Thus, according to the present invention, the registration of patient data and the simulation of the (future) change or growth/shrinking of a specific object within other tissue can be performed in a more simple manner, especially when the using simple or simplified rules according to the present invention (in contrast to solving a biomechanical model of soft tissue deformation, as in the prior art), which provides the advantage that the inventive methods can be performed more rapidly and in case of iteratively applying the inventive data processing methods, the accuracy of a registration or simulation can be improved.

This is especially true when comparing the invention to solving a biomechanical model. If the tumor growth is simulated simply radially away from a seed sitting in the tumor center, without solving a biomechanical model, the present invention is more accurate, since more information about tumor-type, position etc. is used.

### BRIEF DESCRIPTION OF THE DRAWINGS

The invention will be described in more detail by reference to an exemplary embodiment, wherein:
- Figure 1: shows an example of a data processing method for registering (pathological) patient data to (non-pathological) atlas data; and
- Figure 2: shows simple-rules for simulating the growth of a tumor.

### DETAILED DESCRFIPTION OF THE INVENTION

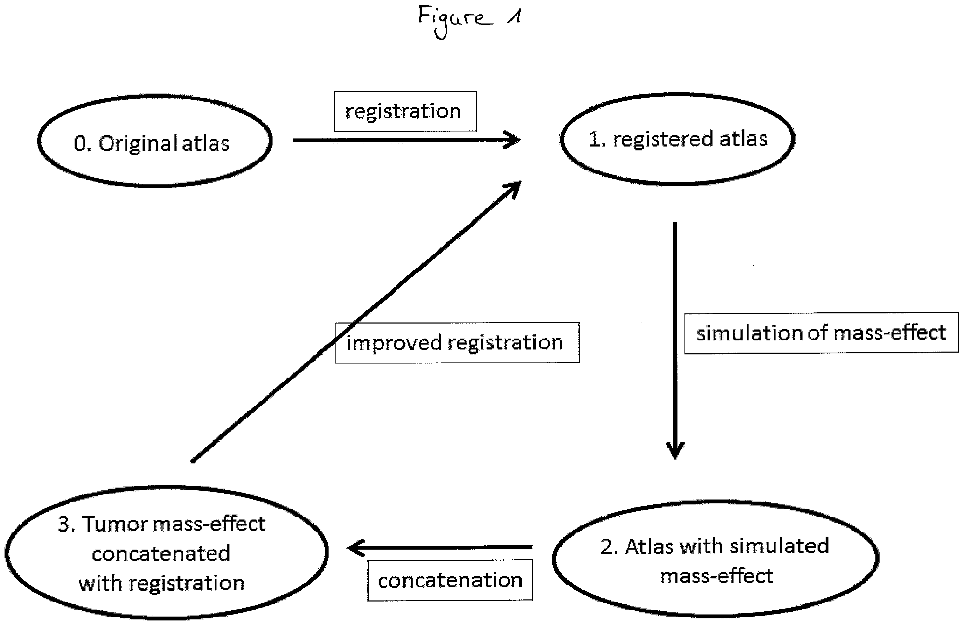

The workflow for the registration of a pathological patient image (not shown in Figure 1) includes in general the simulation of the tumor mass-effect, which simulation is added as an independent step to an (iterative) elastic registration of a patient image to a brain atlas.

The tumor is segmented in the patient image using for example a fuzzy c-means algorithm. After a rigid fusion of the patient image and the atlas (see item O. Original atlas), a first elastic fusion step is performed, to obtain a registered atlas (see item 1.) at the end of which the tumor mass-effect is simulated to obtain an atlas with a simulated mass-effect (see item 2.). The mapping corresponding to the mass-effect is then concatenated with the registration mapping (see item 3.). Subsequently, a new improved registration is performed, in which the tumor is masked out such that it is does not contribute to the similarity measure used to determine the registration mapping. The elastic fusion and tumor simulation steps can be repeated until a desired predefined accuracy is reached.

The tumor mass-effect is computed as follows:
At the end of every iterative registration step, the tumor shown in Fig. 1 is transferred to the atlas using the current registration, and is intersected with a mask for the sub-tentorial region. The anatomical information contained in the atlas is used to define a region of parallel shift of the tissue (which will be referred to as "parallel region"), representing the effect of small tumors. Inside this region, the tissue will be mapped away from the Dura Mater along a reference direction, whereas outside of the parallel region the mapping points radially away from a reference point on the Dura Mater. The determination of the algorithm parameters (parallel region, reference direction, and reference point) is described in the following.

The parallel region is a set of voxels lying within a certain distance to the intersection of the tumor with the Dura Mater.

The reference point is determined as the middle point of the intersection of the tumor with the Dura Mater, with the additional constraint that its distance to the bony structure in front of the brainstem does not exceed a certain value. If this constraint cannot be met, this is an indication for the tumor not lying in the frontal region of the brainstem, and the constraint it is ignored.

The reference direction is the direction from the reference point to the tumor center. The above-mentioned constraint in the determination of the reference point assures that the tendency of tumors to push the brainstem backwards is taken care of.

If the tumor does not touch the Dura Mater, the tissue is shifted radially away from its center. For every tumor the displacement field is set to zero close to the Dura Mater and is smoothed with a Gaussian kernel to avoid discontinuities.

Optionally, the best reference direction can be determined by a simple optimization procedure, choosing amongst a set of proposal directions the one yielding the best similarity measure between the registered patient image and the atlas.

A simple model for the relaxation of the brainstem after tumor resection is given by inverting the mapping of the tumor mass effect, and by multiplying the resulting mapping with a factor between 0 and 1. This takes into account the fact that usually the brainstem does not fully relax to its original position immediately after the resection.

An example for rules for simulating an object depending on the tissue-class (e.g. specified in an atlas) is given in the below table:

| *Tissue class* | *(Simple) rules for stimulating the changes or growth of an object* |
|---|---|
| Liquids (e.g. cerebrospinal fluid, blood) | The object grows into the cavity filled with fluid without encountering resistance: the part of the fluid-filled volume covered by the pathological object simply gets replaced by the pathological object without affecting the surrounding tissue (the object does not exert any pressure on surrounding tissue). |
| | This rule holds as well, if the object that pushes into the fluid region is another tissue class of the atlas (e.g. white matter which is pushed into the fluid by a pathological object in the white matter) |
| White matter, gray matter | The tissue gets pushed away by the object according to the rules specified in Figure 2 |
| Fat, muscles | The tissue gets pushed away from the object radially from the object's center |
| Hard surface (Bone, Dura Mater) | Cannot be pushed away, i.e. represent a hard boundary for simulating the object. However, certain objects penetrate / infiltrate the hard surface (e.g. tumor starting in the bone / in Dura Mater) |

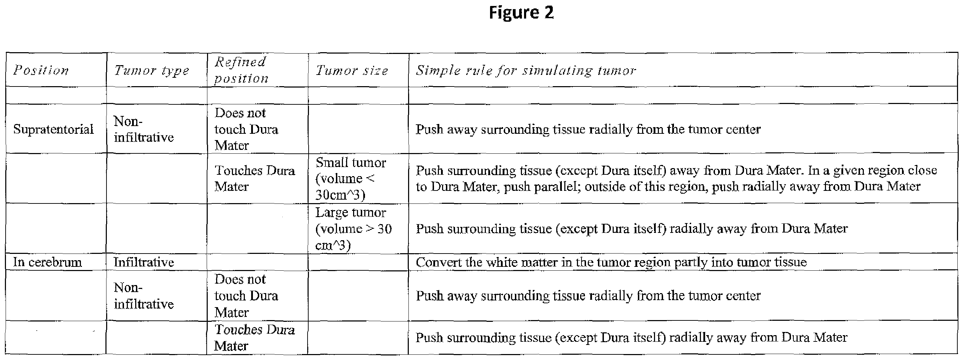

Figure 2 shows a flow-chart illustrating rules for simulating the growth of a tumor being an embodiment of the simulating step.

In a first step, the position of the tumor in the brain is determined. If the tumor is within the cerebrum, it is determined in the next step whether or not the tumor type is infiltrative. In case of an infiltrating tumor, the simple rule for simulating the growth of the tumor is to convert the white matter in the tumor region partly into tumor tissue. In case of a non-infiltrative tumor, the position has to be refined. If the tumor does not touch the Dura Mater, the simple rule for simulating the tumor growth is to push away surrounding tissue radially from the tumor center. In case the tumor touches the Dura Mater, the simple rule for simulating the tumor is to push the surrounding tissue (except the Dura Mater itself) radially away from the Dura Mater.

In case of a sub-tentorial or infratentorial position of the tumor, it is determined whether the tumor type is infiltrative. In case of an infiltrative subtentorial tumor, the gray/white matter is simply changed (at least in part) to being tumor tissue. In case of a non-infiltrative tumor, the position has to be refined. If the tumor does not touch the Dura Mater, the simple rule for simulating the tumor is to push away the surrounding tissue radially from the tumor center. If the tumor touches the Dura Mater, a distinction is made depending on the tumor size. In case of a small tumor (estimated to have a volume below 30 cm³), the simple rule for simulating the tumor is to push the surrounding tissue (except the Dura Mater itself) away from the Dura Mater. In a given region close to the Dura Mater, e.g. having 0-5 mm distance from the Dura Mater, the tumor is pushing surrounding tissue parallel and outside of this region, the tumor pushes surrounding tissue radially away from the Dura Mater. In case of a large tumor (having an estimated volume being above 30 cm³), the simple rule for simulating the simple rule for simulating the tumor is to push the surrounding tissue (except the Dura itself) radially away from the Dura Mater.

Below shown table exemplifies other simple rules for simulating the change or growth of an object.

| *Object* | *(Simple) rule for simulating the object* |
|---|---|
| Catheter in the bladder | Push away surrounding tissue (except for fluid) radially from the center of the bladder |
| Full bladder | Push away tissue surrounding the bladder radially from the center of the bladder |
| Edema | Infiltrating object: replace the tissue in the region of the edema partly by the fluid causing the edema and push surrounding tissue (e.g. skin) slightly away from the center of the edema |
| Contrasting agent | Simulating the dispersion of contrasting agent with time: Contrasting agent does not penetrate the surface of the blood vessels, but moves along the blood vessel => move forward the contrasting agent in time away from the injection point along the vessels |
| Breathing | Move the diaphragm and all tissue close to it up and down according to breathing phase. |
| Brain-shift from opening the skull in brain surgery | Detect the cavity from the brain-shift in the patient image. Push the white and gray matter away from Dura Mater on the full length of the cavity. |
| Non-infiltrating tumor in Spinal Canal | Use the same rules as for tumors in the subtentorial region |
| Aging process of ventricles | Linearly increase the volume of the lateral ventricles as a function of the patient's age, under the constraint that the growth rate in forward and backward direction (towards the face and the back of the head) is larger (e.g. twice as fast) than in the other directions |

## Claims

1. A data processing method for simulating the change or growth of a specific object data describing or representing a location within normal patient data, the method comprising the following steps performed by a computer:
a) acquiring the patient data which comprises anatomical information of the patient including normal patient data and the specific object data of the patient;
b) segmenting the specific object data in the patient data;
c) acquiring atlas data;
d) acquiring meta-information about components of the atlas data;
e) performing a fusion of the acquired patient data excluding the segmented specific object data and the atlas data to obtain registered atlas data; and
f) simulating the growth of a simulated specific object within the registered atlas data using the registered atlas data and the meta-information about components of the atlas data being adjacent to the area of the specific object,
wherein in the simulation step f) a set of rules for simulating the change or growth of the specific object is used, the rules being preferably obtained from a statistical analysis of a number of different patients having been observed during a time period covering a process of change or growth of a specific object or tumor in tissue,
wherein a rule is associated as meta-information to specific tissue or tissue-classes or specific relative locations and/or to the type of the specific object;
wherein the meta-information includes an information of the probability for a predefined rule for simulating the change or growth of the specific object to occur.

2. The data processing method of claim 1, wherein the specific object data describes or relates to a pathological structure or a tumor.

3. The data processing method according to one of the preceding claims, wherein the fusion step e) includes a rigid fusion and/or an elastic fusion.

4. The data processing method according to one of the preceding claims, wherein the simulated grown specific object effects, during or at the end of simulating the growth, a change in surrounding tissue data, such as a transformation of at least a part of the surrounding tissue data into specific object data or tumor tissue data and/or a shift of the position of at least a part or all of the surrounding tissue data.

5. The data processing method according to one of the preceding claims, wherein the simulated grown specific object is segmented in the registered atlas data after the end of the growth simulation to obtain segmented registered atlas data and the following steps are repeated, preferably until a desired accuracy is reached:
g) performing a fusion of the segmented registered atlas data excluding the segmented specific object data and the registered atlas data to obtain further registered atlas data;
h) simulating the growth of a simulated specific object within the further registered atlas data using the further registered atlas data and the meta-information about components of the further atlas data being adjacent to the area of the specific object to obtain still further registered atlas data containing a simulated grown specific object.

6. A data processing method for registering patient data containing normal patient data and specific object data to atlas data not containing this specific object data, the method comprising the steps of claim 1 to obtain registered atlas data containing a simulated grown specific object.

7. A program which, when running on a computer or when loaded onto a computer, causes the computer to perform the method steps of the method according to one of the preceding claims.

8. A program storage medium on which the program of the previous claim is stored.

9. A computer on which the program of claim 7 is running or into the memory of which the program is loaded.

10. A signal wave, in particular a digital signal wave, carrying information which represents the program of claim 7.

11. A medical image processing system, comprising:
an analytical device for generating or delivering patient data or patient images of a patient; and
a computer constituted to perform the method of one of claims 1 to 6.

## Patentansprüche

1. Datenverarbeitungsverfahren zum Simulieren der Veränderung oder des Wachstums von spezifischen Objektdaten, die eine Stelle innerhalb von normalen Patientendaten beschreiben und darstellen, wobei das Verfahren die folgenden Schritte umfasst, die von einem Computer durchgeführt werden:
a) Erfassen der Patientendaten, die anatomische Informationen des Patienten umfassen, einschließlich normaler Patientendaten und der spezifischen Objektdaten des Patienten;
b) Segmentieren der spezifischen Objektdaten in den Patientendaten;
c) Erfassen von Atlasdaten;
d) Erfassen von Metainfonnationen über Komponenten der Atlasdaten;
e) Durchführen einer Zusammenführung der erfassten Patientendaten, ausschließlich der segmentierten spezifischen Objektdaten und der Atlasdaten, um registrierte Atlasdaten zu erhalten; und
f) Simulieren des Wachstums eines simulierten spezifischen Objekts innerhalb der registrierten Atlasdaten unter Verwendung der registrierten Atlasdaten und der Metainformationen über Komponenten der Atlasdaten, die an den Bereich des spezifischen Objekts angrenzen,
wobei in dem Simulationsschritt f) eine Reihe von Regeln zum Simulieren der Veränderung oder des Wachstums des spezifischen Objekts verwendet wird, wobei die Regeln vorzugsweise durch eine statistische Analyse von mehreren verschiedenen Patienten erhalten werden, die über einen Zeitraum beobachtet wurden, der einen Veränderungs- oder Wachstumsvorgang des spezifischen Objekts oder Tumors im Gewebe abdeckt,
wobei eine Regel als Metainformation mit spezifischem Gewebe oder Gewebeklassen oder spezifischen relativen Stellen und/oder dem Typ des spezifischen Objekts in Verbindung steht;
wobei die Metainformationen eine Information über die Wahrscheinlichkeit eines Eintretens einer vordefinierten Regel zum Simulieren der Veränderung oder des Wachstums des spezifischen Objekts enthält.

2. Datenverarbeitungsverfahren nach Anspruch 1, wobei die spezifischen Objektdaten eine pathologische Struktur oder einen Tumor beschreiben oder sich darauf beziehen.

3. Datenverarbeitungsverfahren nach einem der vorstehenden Ansprüche, wobei der Zusammenführungsschritt e) eine starre Zusammenführung und/oder eine elastische Zusammenführung enthält.

4. Datenverarbeitungsverfahren nach einem der vorstehenden Ansprüche, wobei das simulierte gewachsene spezifische Objekt während oder am Ende des Simulierens des Wachstums eine Veränderung in umgebenden Gewebedaten bewirkt, wie z. B. eine Transformation von mindestens einem Teil der umgebenden Gewebedaten in spezifische Objektdaten oder Tumorgewebedaten und/oder eine Verschiebung der Position von mindestens einem Teil der oder allen umgebenden Gewebedaten.

5. Datenverarbeitungsverfahren nach einem der vorstehenden Ansprüche, wobei das simulierte gewachsene spezifische Objekt in den registrierten Atlasdaten nach dem Ende der Wachstumssimulation segmentiert wird, um segmentierte registrierte Atlasdaten zu erhalten, und die folgenden Schritte wiederholt werden, vorzugsweise bis eine gewünschte Genauigkeit erreicht ist:
g) Durchführen einer Zusammenführung der segmentierten registrierten Atlasdaten, ausschließlich der segmentierten spezifischen Objektdaten und der registrierten Atlasdaten, um weitere registrierte Atlasdaten zu erhalten;
h) Simulieren des Wachstums eines simulierten spezifischen Objekts innerhalb der weiteren registrierten Atlasdaten unter Verwendung der weiteren registrierten Atlasdaten und der Metainfonnationen über Komponenten der weiteren Atlasdaten, die an den Bereich des spezifischen Objekts angrenzen" um noch weitere registrierte Atlasdaten zu erhalten, die ein simuliertes gewachsenes spezifisches Objekt enthalten.

6. Datenverarbeitungsverfahren zum Registrieren von Patientendaten, die normale Patientendaten und spezifische Objektdaten bis Atlasdaten enthalten, die diese spezifischen Objektdaten nicht enthalten, wobei das Verfahren die Schritte nach Anspruch 1 umfasst, um registrierte Atlasdaten zu erhalten, die ein simuliertes gewachsenes spezifisches Objekt enthalten.

7. Programm, das, wenn es auf einem Computer läuft oder wenn es auf einen Computer geladen wird, den Computer veranlasst, die Verfahrensschritte des Verfahrens nach einem der vorstehenden Ansprüche durchzuführen.

8. Programmspeichermedium, auf dem das Programm des vorigen Anspruchs gespeichert ist.

9. Computer, auf dem das Programm nach Anspruch 7 läuft oder auf dessen Speicher das Programm geladen ist.

10. Signalwelle, insbesondere eine digitale Signalwelle, die Informationen trägt, die das Programm nach Anspruch 7 darstellen.

11. Medizinisches Bildverarbeitungssystem, umfassend:
eine analytische Vorrichtung zum Erzeugen oder Übermitteln von Patientendaten oder Patientenbildern eines Patienten; und
einen Computer, der eingerichtet ist, um das Verfahren nach einem der Ansprüche 1 bis 6 durchzuführen.

## Revendications

1. Procédé de traitement de données destiné à simuler des données de changement ou de croissance d'un obj et spécifique décrivant ou représentant un emplacement dans des données normales de patient, le procédé comprenant les étapes suivantes réalisées par un ordinateur :
a) acquisition des données de patient qui comprennent des informations anatomiques du patient comprenant des données normales de patient et les données d'objet spécifique du patient ;
b) segmentation des données d'objet spécifique dans les données du patient ;
c) acquisition de données d'atlas ;
d) acquisition de méta-informations sur des composantes des données d'atlas ;
e) réalisation d'une fusion des données de patient acquises à l'exclusion des données d'objet spécifique segmentées et des données d'atlas pour obtenir des données d'atlas enregistrées ; et
f) simulation de la croissance d'un objet spécifique simulé à l'intérieur des données d'atlas enregistrées à l'aide des données d'atlas enregistrées et des méta-informations sur les composantes des données d'atlas étant adjacentes à la zone de l'objet spécifique,
dans lequel dans l'étape de simulation f) une série de règles destinées à simuler le changement ou la croissance de l'objet spécifique est utilisée, les règles étant de préférence obtenues à partir d'une analyse statistique d'un nombre de patients différents ayant été observés pendant une période de temps couvrant un processus de changement ou de croissance d'un objet spécifique ou d'une tumeur dans un tissu,
une règle étant associée en tant que méta-information à un tissu spécifique ou à des classes de tissu spécifiques ou des emplacements relatifs spécifiques et/ou au type d'objet spécifique ;
les méta-informations comprenant une information de la probabilité qu'une règle prédéfinie simule le changement ou la croissance de l'objet spécifique.

2. Procédé de traitement de données selon la revendication 1, dans lequel les données d'objet spécifique décrivent ou sont en rapport avec une structure pathologique ou une tumeur.

3. Procédé de traitement de données selon l'une des revendications précédentes, dans lequel l'étape de fusion e) comprend une fusion rigide et/ou une fusion élastique.

4. Procédé de traitement de données selon l'une des revendications précédentes, dans lequel l'objet spécifique grossi simulé effectue, pendant ou à la fin de la simulation de la croissance, un changement de données de tissu environnant, tel qu'une transformation d'au moins une partie des données de tissu environnant en données d'objet spécifique ou données de tissu tumoral et/ou un décalage de position d'au moins une partie ou de la totalité des données de tissu environnant.

5. Procédé de traitement de données selon l'une des revendications précédentes, dans lequel l'objet spécifique grossi simulé est segmenté en données d'atlas enregistrées après la fin de la simulation de croissance pour obtenir des données d'atlas enregistrées segmentées et les étapes suivantes sont répétées, de préférence jusqu'à ce qu'on obtienne une précision désirée :
g) réalisation d'une fusion des données d'atlas enregistrées segmentées à l'exclusion des données d'objet spécifique segmentées et des données d'atlas enregistrées pour obtenir des autres données d'atlas enregistrées ;
h) simulation de la croissance d'un objet spécifique simulé parmi les autres données d'atlas enregistrées à l'aide des autres données d'atlas enregistrées et des méta-informations sur les composantes des autres données d'atlas étant adjacentes à la zone de l'objet spécifique pour obtenir encore d'autres données d'atlas enregistrées contenant un objet spécifique grossi simulé.

6. Procédé de traitement de données destiné à enregistrer des données de patient contenant des données normales de patient et des données d'objet spécifique dans des données d'atlas ne contenant pas ces données d'objet spécifique, le procédé comprenant les étapes de la revendication 1 pour obtenir des données d'atlas enregistrées contenant un objet spécifique grossi simulé ayant.

7. Programme qui, lorsqu'il est exécuté sur un ordinateur ou lorsqu'il est chargé sur un ordinateur, amène l'ordinateur à réaliser les étapes de procédé du procédé selon l'une des revendications précédentes.

8. Support de stockage de programme sur lequel le programme de la revendication précédente est stocké.

9. Ordinateur sur lequel le programme de la revendication 7 est exécuté ou dans la mémoire duquel le programme est chargé.

10. Onde de signal, en particulier onde de signal numérique, transportant des informations qui représentent le programme de la revendication 7.

11. Système de traitement d'image médicale, comprenant :
un dispositif analytique destiné à générer ou fournir des données de patient ou des images de patient d'un patient ; et
un ordinateur conçu pour effectuer le procédé selon l'une des revendications 1 à 6.
